# EUROPEAN PATENT SPECIFICATION

(11) **EP 1 508 322 B1**
(45) Date of publication and mention of the grant of the patent: **04.10.2006**
(21) Application number: 03755265.0
(22) Date of filing: 07.04.2003
(51) Int. Cl.: A61K 8/11, A61K 8/37, A61Q 5/00, A61Q 19/00

(54) **POWDER SURFACE-TREATED WITH SPECIFIC HALF ESTER OIL AND COSMETIC COMPOSITION CONTAINING THE SAME**
MIT EINEM SPEZIELLEN HALBESTERÖL OBERFLÄCHENBEHANDELTES PULVER UND DIESES ENTHALTENDE KOSMETISCHE ZUSAMMENSETZUNG
SURFACE DE POUDRE TRAITEE AVEC UNE HUILE D'HEMIESTER SPECIFIQUE ET COMPOSITION COSMETIQUE LA CONTENANT

(30) Priority: 27.05.2002 JP 2002153014; 25.02.2003 JP 2003047064
(43) Date of publication of application: 23.02.2005
(73) Proprietor: Miyoshi Kasei, Inc., Saitama-shi, Saitama 337-0975 (JP); The Nisshin OilliO Group, Ltd., Tokyo 104-8285 (JP)
(72) Inventor: HASEGAWA, Yukio c/o R & D Dept., MIYOSHI KASEI INC, Saitama-shi, Saitama 337-0975 (JP); TAKAHASHI,Hideki c/o R & D Dept. MIYOSHI KASEI INC, Saitama-shi, Saitama 337-0975 (JP); HISHIKAWA, Noboru c/o Yokohama Isogo Adm. Dept., Isogo-ku Yokohama-shi, Kanagawa 235-8558 (JP); YAMATO, Yoshihito c/o Yokohama Isogo Adm. Dept., Isogo-ku Yokohama-shi, Kanagawa 235-8558 (JP)
(74) Representative: Santarelli
(86) International application number: PCT/JP2003/004379
(87) International publication number: WO 2003/099239

(56) References cited:
- FR-A- 2 795 949
- GB-A- 1 489 493
- JP-A- 6 031 152
- JP-A- 7 207 178
- JP-A- 2001 279 129
- JP-A- 2002 179 941
- PATENT ABSTRACTS OF JAPAN vol. 2002, no. 07, 3 July 2002 (2002-07-03) & JP 2002 080748 A (MIYOSHI KASEI KK), 19 March 2002 (2002-03-19)
- PATENT ABSTRACTS OF JAPAN vol. 2002, no. 02, 2 April 2002 (2002-04-02) & JP 2001 279129 A (KANEBO LTD), 10 October 2001 (2001-10-10)

## Description

### TECHNICAL FIELD

The present invention relates to a novel surface-treated powder (base). More specifically, the invention relates to a surface-treated powder coated with a half ester oil containing a specific half ester compound as well as a cosmetic composition blended with the surface-treated powder. The surface-treated powder of the invention has excellent adhesion to skin and hair, great spreadability on skin and hair, good dispersibility of the surface-treated powder itself, highly excellent dispersibility thereof in general oils such as ester oils and triglycerides and in silicone-series or fluorine-series oils, and has a very moist feel. Cosmetics blended with such surface-treated powder can get great improvement of feel on use, makeup retention, and product stability over time. Additionally because the coated powder has highly excellent dispersibility in oils and fats, the powder is applicable to not only cosmetics but also as powders for use in the fields of, for example, additives for plastics, inks, paints and toners.

### BACKGROUND OF THE INVENTION

Cosmetics to be blended with powders have included so far, for example, makeup cosmetics, skin care cosmetics, body care cosmetics, hair care cosmetics, and sun screening cosmetics. More specifically, the compositions of cosmetics blended with powders are broadly divided into powders, oil components, active ingredients, and aqueous components. Depending on the ratio of these components, there are formulations such as powder type, cream type and liquid type. The types of the powders include inorganic powders such as extender pigment, white pigment, coloring pigment and pearl, and organic powders such as nylon, PMMA, acylated lysine and tar dye. These powders are appropriately selected and blended, depending on the type of cosmetic to be blended and the purpose of the cosmetic. At the process of producing a product, such powder is mixed and dispersed in a small amount of oily agents and a trace amount of other additives. Otherwise, a small amount of powder is added to and dispersed in oily agents. Additionally for a formulation of emulsion type, water is mixed for emulsification. Functions demanded for such powders during the production process are dispersibility in oily agents and stability after dispersion. Additionally, powders to be blended in the cosmetics are for the purpose of improving the feel on use (giving sliding property) , providing tone and gloss, giving adhesiveness to skin and hair, providing protection against ultraviolet ray and infrared ray, preventing the removal of makeup and the like.

So as to give such functions to powders, techniques for surface treatment have been proposed, including for example surface treatment with silicone compounds, surface treatment with fluorine compounds, surface treatment with fatty acids, surface treatment with acylated amino acids and acylated peptides, and surface treatment with lecithin, ceramide, alkyl silane and alkyl titanate. FR-A-2 795 949 discloses surface treated powders for cosmetics coated with a first solid layer containing at least one compound selected from reactive organopolysiloxane, polyolefin, hydrogenated lecitin, N-acyl amino acid, fatty acid or dextrin fatty acid ester, and a second coating layer containing at least one compound selected from organopolysiloxane, modified alkylsilane or a branched fatty acid. For example, powders surface-treated with silicone compounds and fluorine compounds have excellent water repellent effect without stickiness, but these powders have poor adhesion to skin and hair, and poor dispersibility in general oils such as ester oils and triglycerides, as well as poor moist feeling. The powders surface-treated with fatty acids have good adhesion to skin and hair, but have very poor aesthetics with respect to smooth spreadability and extension, and have poor dispersibility in general oils such as ester oils and triglycerides or in silicone-series oils or fluorine-series oils, so that the powders have less moist feeling. Although powders surface-treated with acylated amino acids or ceramide have excellent adhesion to skin and hair with moist feeling, the powders lack smooth feeling and are poor in terms of dispersibility in oil. Powders surface-treated with lecithin have a very smooth feeling, great adhesion to skin and hair and have a moist feel, but the powders are poorly dispersible in general oils such as ester oils and triglyceride, and silicone-series or fluorine-series oils. Powders treated with alkyl silane and alkyl titanate have smooth feeling on use and great dispersibility in general oils such as ester oils and triglycerides, silicone-series oils or fluorine-series oils. However, the powders have poor adhesion to skin and hair and lack much moist feeling.

As described above, various techniques for surface treatments have been proposed. However, there is not yet any surface-treated powder capable of simultaneously exerting the four characteristic features, namely great adhesion to skin and hair, good spreadability on skin and hair, high dispersibility in general oils such as ester oils and triglycerides, silicone-series oils and fluorine-series oils and moist feeling at sufficiently high levels. Therefore, further improvement in this field has strongly been desired.

### DISCLOSURE OF THE INVENTION

Accordingly, the inventors made investigations so as to overcome the problems described above. The inventors found that a powder coated with an ester oil comprising an ester compound having carbon atoms of a certain total number or more as obtained by reacting various alcohols with carboxylic acids and having an acid value of a certain value or more, had excellent adhesion to and spreadability on skin and hair as well as great dispersibility and moist feel. Thus, the invention has been achieved.

In other words, the invention provides a surface-treated powder being coated with a half ester oil, wherein the half ester oil comprises an ester compound having 16 or more carbon atoms in total, as can be obtained by reacting one or more alcohols having one to 36 carbon atoms with one or more carboxylic acids having one to 36 carbon atoms, and wherein the half ester oil has an acid value of 15 or more.

In a preferable embodiment of the invention, the alcohol is monovalent or polyvalent alcohol comprising at least one selected from the group consisting of linear carbons, branched carbons, saturated carbons, unsaturated carbons, alicyclic carbons and aromatic rings, while the carboxylic acid is monobasic acid or polybasic acid comprising at least one selected from the group consisting of linear carbons, branched carbons, saturated carbons, unsaturated carbons, alicyclic carbons and aromatic rings, or hydroxylic acid thereof.

In a more preferable embodiment of the invention, the acid value of the ester oil is 15 or more to 100 or less, while the ratio by weight between a powder to be treated by coating and an agent for treating the surface is 100 : 0.1 to 50.

In a different aspect of the invention, there is provided a cosmetic containing the surface-treated powder at 0.1 to 100 % by weight.

### BEST MODE FOR CARRYING OUT THE INVENTION

The constitution of the invention is now described below.

The ester oil for use in coating a powder in accordance with the invention contains ester compounds having 16 or more carbon atoms in total, as obtained by partially esterifying an alcohol having one to 36 carbon atoms with a carboxylic acid having one to 36 carbon atoms and has an acid value of 15 or more.

The alcohol having one to 36 carbon atoms for use in accordance with the invention includes monovalent linear alcohols such as methanol, ethanol, propanol, butanol, pentanol, hexanol, heptanol, octanol, nonanol, decanol, undecanol, dodecanol (lauryl alcohol), tridecanol, tetradecanol (myristyl alcohol), pentadecanol, hexadecanol (cetyl alcohol), heptadecanol, octadecanol (stearyl alcohol), nonadecanol, eicosanol (aralkyl alcohol), docosanol (behenyl alcohol), tetracosanol, and setostearyl alcohol; monovalent branched alcohols such as isopropyl alcohol, isobutyl alcohol, isopentyl alcohol, isohexanol, isoheptanol, iso-octanol, dimethyloctanol, isononanol, isodecanol, isoundecanol, isododecanol, isotridecanol, isotetradecanol, isopentadecanol, isohexadecanol (hexyldecanol), isoheptadecanol, isooctadecanol (isostearyl alcohol), isononadecanol, isoeicosanol (octyldodecanol), 2-ethylhexanol, 2-butyloctanol, 2-hexyldecanol, 2-octyldodecanol, 2-decyltetradecanol, 2-dodecylhexadecanol, 2-tetradecyloctadecanol, and 2-hexadecyloctadecanol; monovalent unsaturated alcohols such as undecenol, laurolenol, myristolenol, palmitolenol, oleyl alcohol, elaidyl alcohol, linoleyl alcohol, linolenyl alcohol, erucyl alcohol, brassidyl alcohol, arachidyl alcohol, and jojova alcohol; sterols such as cholesterol, dihydrocholesterol, desmosterol, lanosterol, dihydrolanosterol, agnosterol, latosterol, sitosterol, campesterol, stigmasterol, brassicasterol, ergosterol, phytosterol, and lanolin alcohol; divalent or higher valent alcohols such as ethylene glycol, propylene glycol, trimethylene glycol, butanediol, pentanediol, hexanediol, heptanediol, octanediol, nonanediol, decanediol, pinacol, hydrobenzoin, benzpinacol, cyclopentane-1,2-diol, cyclohexane-1,2-diol, cyclohexane-1,4-diol, dimer diol, hydrogenated dimer diol, neopentylglycol, glycerin, trimethylol propane, trimethylol propane condensate, trimethylol ethane, pentaerythrit, pentaerythrit condensate, sorbit, glycerin condensate, polyethylene glycol, and polypropylene glycol. In accordance with the invention, these may be used singularly or in mixture of appropriate combinations thereof.

As the carboxylic acid having one to 36 carbon atoms (n = 1-3) for use in accordance with the invention, any such carboxylic acid of saturated, unsaturated, hydroxy, aromatic type and the like may be used. For example, monobasic acid (n = 1) includes for example linear fatty acids such as formic acid, acetic acid, propionic acid, lactic acid, caproic acid, caprylic acid, capric acid, undecanoic acid, lauric acid, tridecanoic acid, myristic acid, pentadecanoic acid, palmitic acid, margaric acid, stearic acid, nonadecanoic acid, arachidic acid, behenic acid, cerotic acid, montanic acid, and melissic acid; unsaturated fatty acids such as undecylenic acid, palmitoleic acid, oleic acid, linoleic acid, linolenic acid, and arachidonic acid; branched fatty acids such as isooctyl acid (2-ethylhexanoic acid), neotridecanoic acid, isomyristic acid, isopalmitic acid and isostearic acid; and hydroxyfatty acids such as 12-hydroxystearic acid and ricinolic acid. Polybasic acids (n = 2-3) include for example oxalic acid, malonic acid, succinic acid, cyclobutane-1,1-dicarboxylic acid, cyclohexane-1, 2-dicarboxylic acid, phenylene-1,2-diacetic acid, diglycolic acid, dithioglycolic acid, glutaric acid, adipic acid, pimeric acid, suberic acid, azelaic acid, sebacic acid, undecandionoic acid, dodecanedioic acid, eicosandioic acid, octacosandioic acid, 1-, 10-decamethylenedioic acid, 1-, 12-dodecamethylenedioic acid, 1-, 15-pentadecamethylenedioic acid, 1-,28-octacosamethylenedioic acid, 7-ethyloctadecanedioic acid, dimer acid, and hydrogenated dimer acid. In accordance with the invention, the "dimer acid" means dibasic acid generated by dimerization of an unsaturated fatty acid with 11 to 22 carbon atoms. The unsaturated dibasic acid includes for example fumaric acid, maleic acid and itaconic acid. The hydroxy-polybasic acid includes for example tartaric acid, malic acid, mucic acid, and citric acid. As the hydroxy-polybasic acid, additionally, amino acids and acylated amino acids may also be used. In accordance with the invention, these may be used singularly or in mixture of an appropriate combination thereof.

The partial ester in accordance with the invention is a partial ester between the alcohol and the carboxylic acid and can be synthetically prepared by esterification. The esterification can be carried out by charging the individual raw materials in an appropriate reaction container and then reacting them together in the presence or absence of an acid, an alkali or a metal catalyst, preferably in an organic solvent or/and a gas inert to the reaction, at 150 to 200 °C for several hours to about 10 hours, while the byproduct water is eliminated. In case that a catalyst is used, herein, the catalyst is added to 0. 001 to 1. 0 % of the weight of the reactants. After the completion of the reaction, the product may contain unreactive materials, which are separated and removed by known methods such as rinsing in water, elimination of acids with alkalis, and treatment with adsorbents such as silica gel and are then purified additionally by decoloring and eliminating the odor. The half ester oil thus obtained is in a liquid or solid state at ambient temperature. Preferably, the half ester oil in accordance with the invention has an acid value of 15 or more and contains an ester compound with 16 or more carbon atoms in total. So as to get the effect of the surface-treated powder of the invention, more preferably, the acid value of the half ester oil is 15 or more to 100 or less. In view of the feeling of the treated powder, the ester oil then is preferably in liquid, semi-solid or solid with plasticity state at ambient temperature. More preferably, the ester oil is in liquid or in semi-solid state, specifically with a melting point of 70 °C or less. Taking account of adhesion to skin and dispersibility in other oils, preferably, the ester oil is a compound obtained by reaction of one or more branched or unsaturated alcohols with mono valence to six valence and one or more dibasic acids or tribasic acids as a preferable combination of reactants, and has ester-binding sites of 6 or less in the structure of one molecule.

When the acid value of the ester oil is less than 15, the adsorptivity thereof to a powder base is poor, so that the half ester oil is not used for such surface treatment, causing poor adhesion to skin and hair. When the ester compound has less than 16 carbon atoms in total, further, the irritation on skin and hair is strong, unpreferably from a safety standpoint. Additionally, the feel of the resulting surface-treated powder on use, is poor in terms of smoothness and lack of moist feel. Herein, the acid value of the half ester oil in accordance with the invention can be measured by methods known to persons skilled in the art, for example according to "Kijyun Yushi Bunseki Shiken Houho (Analytical Method of Standard Oils and Fats)" (edited by Japan Oil Chemist's Society).

The amount for surface treatment of the ester oil with an acid value of 15 or more, which contains such ester compound with total carbon atoms of 16 or more varies depending on the type and specific surface area of an inorganic powder base. The amount is 0.1 to 50 parts by weight of the powder. Preferably, the amount is 0.3 to 35 parts by weight. Such ester oil for surface treatment may satisfactorily be in mixture of two types or more thereof for the coating treatment. When the amount used for coating is less than 0.1 parts by weight, the surface of the powder particle cannot get a uniformly coated layer so that good adhesion to skin and hair and feel on skin and hair cannot be obtained. When the amount is more than 50 parts by weight, the ester oil simply works to aggregate the powder particle, so that the adhesion to skin and hair or the feeling on use cannot further be improved.

The powder for use in accordance with the invention includes not only powders for cosmetics but also powders for use in individual fields of plastic additives, inks, paints, toners and the like. The mean particle size is preferably about 3,000 µm to 0. 001 µm, more preferably about 200 µm to 0.01 µm. Additionally, the particle size of these powders can be measured as a mean value by a method under observation with optical microscope or electron microscope. The particle size of a particle that is not spherical in shape can be determined as a mean of the total of long diameter, short diameter, thickness and the like.

Inorganic powders, for example, include extender pigments such as mica, sericite, talc, kaolin, synthetic mica, white mica, gold mica, red mica, black mica, lithia mica, calcium carbonate, magnesium carbonate, calcium phosphate, alumina, magnesium oxide, aluminium hydroxide, barium sulfate, magnesium sulfate, silicic acid, silicic anhydride, magnesium silicate, aluminium silicate, magnesium aluminium silicate, calcium silicate, barium silicate, strontium silicate, silicon carbide, metal salts of tungstic acid, magnesium aluminate, magnesium metasilicate aluminate, chlorohydroxyaluminium, clay, bentonite, zeolite, smectite, hydroxyapatite, ceramic powder, boron nitride, and silica; specific composite extender pigments such as Excel Mica, Excel Pearl and Powder La Vie marketed by Miyoshi Kasei, Inc.; white pigments such as titanium dioxide, zinc oxide and cerium oxide; coloring pigments such as red iron oxide, yellow iron oxide, black iron oxide, chromium oxide, chromium hydroxide, Prussian blue, ultramarine blue, inorganic blue pigments, carbon black, low ordertitanium oxides, mango violet, cobalt violet, tar pigment prepared into the form of lake, and naturally occurring dyes prepared into the form of lake; optically gloss pigments such as bismuth oxychloride, titanium mica, pearl essence, powder prepared by covering synthetic mica with titanium dioxide, powder prepared by covering silica flake with titanium dioxide, which is available under the trade name of "Metashine" from Nippon Sheet Glass Co., Ltd., powder prepared by covering alumina flake with tin oxide and titanium dioxide, powder prepared by covering aluminium flake with titanium dioxide, powder prepared by covering Copper Flake available from Eckert Inc. USA with silica, powder prepared by covering bronze flake with silica, and powder prepared by covering aluminium flake with silica; ultrafine powder of a mean particle size less than 0.1 µm such as ultrafine titanium dioxide, ultrafine zinc oxide, ultrafine iron oxide, and ultrafine cerium oxide; powders with specific particle shapes such as butterfly-shaped barium sulfate, petal-shaped zinc oxide, and nylon fiber of a long diameter of several millimeters; other powders such as luminescent powder marketed under the trade name of "Luminova Series" by Mitsui & Co., Ltd., aluminium powder, stainless powder, tourmaline powder, and amber powder; and the like.

Organic powders includes for example wool powder, polyamide powder, polyester powder, polyethylene powder, polypropylene powder, polystyrene powder, polyurethane powder, benzoguanamine powder, polymethyl benzoguanamine powder, tetrafluoroethylene powder, polymethyl methacrylate powder, cellulose powder, silk powder, silicone powder, silicone rubber powder, synthetic resin powders such as styrene-acrylate copolymer, divinylbenzene-styrene copolymer, vinyl resin, urea resin, phenol resin, fluorine resin, silicone resin, acrylic resin, melamine resin, epoxy resin, and polycarbonate resin, microcrystalline fiber powder, starch powder, acylated lysine powder, long-chain alkylphosphate metal salt powder, metal soap powder, CI pigment Yellow, and CI pigment orange. The tar dye includes for example Red No.3, Red No.10, Red No.106, Red No.201, Red No.202, Red No.204, Red No.205, Red No.220, Red No.226, Red No.227, Red No.228, Red No.230, Red No.401, Red No.505, Yellow No.4, Yellow No.5, Yellow No.202, Yellow No.203, Yellow No.204, Yellow No.401, Blue No.1, Blue No.2, Blue No.201, Blue No.404, Green No.3, Green No.201, Green No.204, Green No.205, Orange No.201, Orange No.203, Orange No.204, Orange No.206, and Orange No.207. Natural dyes include powders such as carmine, laccaic acid, carsamine, brazilin, and crocin.

Furthermore, the form of the powder to be used may include general powder forms to be blended in cosmetics, such as mixture and composite forms of plural powders and attached form. For example, these powders are prepared in a composite or in a doped form, if necessary, which are then used. Examples thereof include powder prepared by covering inorganic coloring pigments such as red iron oxide with silicic anhydride, powders prepared by covering nylon with white pigments, and powders prepared by covering extender pigments with ultrafine white pigments.

The method for surface-treating powders with the half ester oil in accordance with the invention includes a step of coating the carboxylic acid moiety as it is in the free form, or a step of treating the carboxylic acid moiety with any of Na, K and polyvalent metals such as Ba, Zn, Ca, Mg, Fe, Zr, Co, Al, and Ti, ammonium or oniums of organic alkanol amines such as monoethanolamine, diethanolamine, triethanolamine, 2-amino-2-methyl-propanol, 2-amino-2-methyl-1,3-propanediol, and triisopropanolamine. Herein, the treatment includes, but is not limited to a surface treatment that is carried out after or simultaneous with substituting the hydrogen atom of the carboxylic acid moiety with another metal or an organic group. Specifically, the treatment includes the following methods. 1. A dry method including a step of mixing a half ester oil with a powder using a Henschel mixer or a super mixer and subsequently drying the resulting mixture. 2. A method including a step of kneading using a kneader or an extruder and subsequent drying. 3. A method including a step of dispersing a powder in water or an organic solvent using a ball mill and a mechano-chemical mill such as a sand grinder and mixing the powder in a half ester oil, and then removing the solvent and drying the resulting powder. 4. A method of making a powder base and a half ester oil in contact to each other in high-speed air stream with for example JET Atomizer for coating the powder. The surface-treatment method herein referred to is not limited to these methods described above, as long as any suchmethod is satisfactorily applicable to the surface treatment of powder base.

Additionally, the half ester oil for the surface treatment in accordance with the invention is very useful as a surface-treating agent for layer A or B as described in JP-A-2001-72527 and JP-A-2002-80748 proposed by the inventors.

So as to improve the affinity and adhesion with a surface-treating agent, the powder base for surface treatment may be coated, for example, with at least one of oxides or hydrated oxides of aluminium, calcium, magnesium, cerium, silicone, zirconium, titanium, zinc, iron, cobalt, manganese, nickel and tin. Further, these powder bases may satisfactorily be powders preliminarily treated with known surface-treating agents for example by surface treatment with silicone compounds, surface treatment with acylated amino acids, surface treatment with fatty acids, surface treatment with fluorine compounds, surface treatment with lecithin, surface treatment with polyethylene, surface treatment with alkylsilane, surface treatment with alkyl titanate, surface treatment with ceramide, and surface treatment with dextrin fatty acid ester in order to enhance effects for surface treatment more.

The amount of the coated powder bases thus obtained in a cosmetic is appropriately selected in a manner dependent on the properties of the cosmetic, but is at 0.1 to 100 % by weight of the entire composition. These surface-treated powders may optionally be mixed with an appropriate amount of one or more types thereof in the cosmetic.

The cosmetic blended with the powder surface-treated with the half ester oil of the invention includes, for example, makeup cosmetics such as powder foundation, liquid foundation, cream foundation, oily foundation, stick foundation, pressed powder, face powder, lipstick, lip gloss, rouge, eye shadow, eyebrow pencil, eye liner, mascara, aqueous nail enamel, oily nail enamel, emulsion type nail enamel, enamel top coat, and enamel base coat; fundamental cosmetics such as emollient cream, emollient lotion, milky lotion, massage lotion, cold cream, whitening cream, emulsion, lotion, aesthetic lotion, carmine lotion, cleansing jell, liquid wash, washing foam, washing cream, washing powder, cleansing cosmetics for makeup, and body gloss; hair cosmetics such as hair gloss, hair cream, hair oil, hair shampoo, hair rinse, hair color, and hair brushing cosmetics; and others including cream and emulsions for sun screening and sun tanning, soap, bathing agents, and fragrance.

Cosmetics blended with half ester oil surface-treated powders in accordance with the invention may appropriately be blended, for example, with pigment dispersants, oils, surfactants, ultraviolet absorbents, preservatives, anti-oxidants, film forming agents, emollient agents, thickeners, dyes, pigments, and fragrance within a range without any deterioration of the advantages of the invention.

### EXAMPLES

The invention is now described in detail in the following Examples, which does not limit the invention in any way.

### [Synthetic Example 1 of half ester oil] (Partial ester of monovalent alcohol and dibasic acid)

In a four-necked flask equipped with a stirrer, a thermometer, a tube for purging nitrogen gas, and a water separator are added 2.2 kg of oleyl alcohol, 0.8 kg of succinic acid, and xylene and p-toluenesulfonic acid at 5 % and 0.2 %, respectively to the whole amount charged. Then, the reaction was carried out at 180 °C until a calculated amount of water accumulates in the water separator. After the completion of the reaction, unreactive substances were separated. According to a general method, the resulting reaction product was deodorized, and was then decolored, to obtain a half ester oil at 2.2 kg. The acid value of the resul ting ester oil was 43.

### [Synthetic Example 2 of half ester oil] (Partial ester of monovalent alcohol and hydroxy-dibasic acid)

In the same manner as in Synthetic Example 1, 2.0 kg of lauryl alcohol was reacted with 1.0 kg of malic acid. After the completion of the reaction, the resulting product was purified by a general method, to obtain a half ester oil at 2.5 kg. The acid value of the resulting ester oil was 35.

### [Synthetic Example 3 of half ester oil] (Partial ester of divalent alcohol and dibasic acid)

In the same manner as in Synthetic Example 1, 1.2 kg of neopentyl alcohol was reacted with 1.8 kg of adipic acid. After the completion of the reaction, the resulting product was purified by a general method, to obtain a half ester oil at 2.4 kg. The acid value of the resulting ester oil was 75.

### [Synthetic Example 4 of half ester oil] (Partial ester of tetravalent alcohol and dibasic acid)

In the same manner as in Synthetic Example 1, 1.0 kg of pentaerythrit was reacted with 2.0 kg of azelaic acid. After the completion of the reaction, the resulting product was purified by a general method, to obtain a half ester oil at 2.4 kg. The acid value of the resulting ester oil was 70.

### [Synthetic Example 5 of half ester oil] (Partial ester of monovalent alcohol and polybasic acid)

In the same manner as in Synthetic Example 1, 2.2 kg of isostearyl alcohol was reacted with 0.8 kg of citric acid. After the completion of the reaction, the resulting product was purified by a general method, to obtain a half ester oil at 2.3 kg. The acid value of the resulting ester oil was 88.

### [Synthetic Example 6 of half ester oil] (Partial ester of monovalent alcohol and polybasic acid)

In the same manner as in Synthetic Example 1, 2.0 kg of isostearyl alcohol was reacted with 1.0 kg of citric acid. After the completion of the reaction, the resulting product was purified by a general method, to obtain a half ester oil at 2.2 kg. The acid value of the resulting ester oil was 135.

### [Synthetic Example 7 of half ester oil] (Partial ester of monovalent alcohol and dimer acid)

In the same manner as in Synthetic Example 1, 1.5 kg of isopalmityl alcohol was reacted with 3.4 kg of dimer acid (dibasic acid with 36 carbon atoms, which contains the dimer of oleic acid as the main component). After the completion of the reaction, the resulting product was purified by a general method, to obtain a half ester oil at 3.6 kg. The acid value of the resulting ester oil was 48.

### [Synthetic Comparative Example 1 of half ester oil] (Partial ester of monovalent alcohol and dibasic acid: acid value of 0.5)

In the same manner as in Synthetic Example 1, 2.5 kg of oleyl alcohol was reacted with 0.5 kg of succinic acid. After the completion of the reaction, the resulting product was purified by a general method, to obtain a half ester oil at 2.1 kg. The acid value of the resulting ester oil was 0.5.

### [Synthetic Comparative Example 2 of half ester oil] (Partial ester of monovalent alcohol and dibasic acid: acid value of 10)

In the same manner as in Synthetic Example 1, 2.3 kg of oleyl alcohol was reacted with 0.7 kg of succinic acid. After the completion of the reaction, the resulting product was purified by a general method, to obtain a half ester oil at 2.3 kg. The acid value of the resulting ester oil was 10.

### [Surface treatment of powder Example 1]

3 parts by weight of the half ester oil of Synthetic Example 1 and light fluid isoparaffin were added to 100 parts by weight of sericite FSE (manufactured by Sanshin Mining Industry Co., Ltd.), for mixing with a high-speed Henschel mixer for 15 minutes. Subsequently, the resulting mixture was passed through an atomizer, and dried at 105 °C for 8 hours to obtain a half ester oil-treated sericite.

### [Surface treatment of powder Example 2]

100 parts by weight of mica M-102 (manufactured by Merck Japan Ltd.,) are added to 500 parts by weight of water, for thorough dispersion with a disper mixer. One part by weight of aqueous 1N NaOH solution is added to 3 parts by weight of the half ester oil of Synthetic Example 2, for saponification of the free carboxylic acid. The saponif iedhalf ester is added to the aqueous dispersion of the powder, for complete dissolution. Aqueous 1 mol/l aluminum sulfate solution is added dropwise until pH reaches 4.5. Then, the dropwise addition is terminated. The resulting mixture is agitated for 15 minutes. After filtration with a centrifuge, the resulting product was dried at 105 °C for 16 hours and ground with an atomizer, to obtain a half ester oil-treated mica.

### [Surface treatment of powder Example 3]

100 parts by weight of talc JA-46R (manufactured by Asada Milling Co., Ltd.) and 60 parts by weight of water are mixed together with a kneader mixer for 5 minutes. 2 parts by weight of the half ester oil of Synthetic Example 3 were added to the resulting mixture, for kneading with a kneader mixture for 15 minutes. The kneaded product was dried at 105 °C for 16 hours and then ground with an atomizer, to obtain a half ester oil-treated talc.

### [Surface treatment of powder Example 4]

100 parts by weight of titanium dioxide CR-50 (manufactured by Ishihara Sangyo Kaisha, Ltd.) and 2.5 parts by weight of the half ester oil of Synthetic Example 4 are mixed together with a Henschel mixer for 10 minutes. The mixture was ground and treated with a jet mill (manufactured by Hosokawa Micron Corporation), and dried at 105 °C for 8 hours, to obtain a half ester oil-treated titanium dioxide.

### [Surface treatment of powder Example 5]

The same surface treatment as in Example 3 was done, except that the talc JA-46R in Example 3 was replaced with yellow iron oxide LL-100P (manufactured by Titan Kogyo Co., Ltd.), to obtain a half ester oil-treated yellow iron oxide.

### [Surface treatment of powder Example 6]

The same surface treatment as in Example 3 was done, except that the talc JA-46R in Example 3 was replaced with red iron oxide R-516P (manufactured by Titan Kogyo Co. , Ltd.), to obtain a half ester oil-treated red iron oxide.

### [Surface treatment of powder Example 7]

The same surface treatment as in Example 3 was done, except that the talc JA-46 in Example 3 was replaced with black iron oxide BL-100P (manufactured by Titan Kogyo Co., Ltd.), to obtain a half ester oil-treated black iron oxide.

### [Surface treatment of powder Example 8]

To 100 parts by weight of ultrafine titanium dioxide MT-100 SA (manufactured by TAYCA Co., Ltd.) are added 5 parts by weight of the half ester oil of Synthetic Example 5, for mixing with a Henschel mixer for 10 minutes. The same surface treatment as in Example 4 was then done, to obtain a half ester oil-treated ultrafine titanium dioxide.

### [Surface treatment of powder Example 9] (Production of MiBrid-treated powder as described in JP-A-2001-72527)

To 100 parts by weight of ultrafine zinc oxide FINEX-50 (manufactured by Sakai Chemical Industry Co., Ltd.) are added 3 parts by weight of silicone oil KF-9901 (manufactured by Shin-etsu Chemical Co., Ltd.), for mixing with a Henschel mixer for 10 minutes. Further, 8 parts by weight of the half ester oil of Synthetic Example 1 are added, for mixing with a Henschel mixer for 10 minutes. The same surface treatment as in Example 4 was then done, to obtain a half ester oil-treated ultrafine zinc oxide.

### [Surface treatment of powder Example 10]

The same surface treatment as in Example 2 was done, except that mica M-102 in Example 2 was replaced with titanium mica (manufactured by Merck Ltd., Japan; TIMIRON SUPER BLUE), to obtain a half ester oil-treated titanium mica.

### [Surface treatment of powder Example 11]

To 100 parts by weight of ultrafine titanium dioxide MT-100SA (manufactured by TAYCA Co., Ltd.) are added 5 parts by weight of the half ester oil of Example 6, for mixing with a Henschel mixer for 10 minutes. The same surface treatment as in Example 4 was then done, to obtain a half ester oil-treated ultrafine titanium dioxide.

### [Surface treatment of powder Example 12]

The same surface treatment as in Example 1 was done, except that sericite FSE in Example 1 was replaced with Powder La Vie (manufactured by Miyoshi Kasei Inc.) and that the half ester oil in Synthetic Example 1 was replaced with the half ester oil of Synthetic Example 7, to obtain a half ester oil-treated Powder La Vie.

### [Comparative Example 1]

Using the ester oil in Synthetic Comparative Example 1, the same processing amounts and the same methods were used for the powders of the individual Examples 1 through 10 and Example 12, to obtain ester oil-treated powders.

### [Comparative Example 2]

Using the ester oil in Synthetic Comparative Example 2, the same processing amounts and the same methods were used for the powders of the individual Examples 1 through 10 and Example 12, to obtain ester oil-treated powders.

### [Comparative Example 3] (Surface treatment with silicone compound)

Using the silicone oil KF-9901 (manufactured by Shin-etsu Chemical Co., Ltd.), the same processing amounts as in the Examples for the powders of Examples 1 through 10 and Example 12 were treated. The processing method is described below. The powders were charged in a kneader under reduced pressure, and a solution of silicone diluted in 50 parts by weight of toluene was added to and mixed with the powders. While the pressure was reduced, toluene was distilled off. Subsequently, the mixtures were agitated at 105 °C for 4 hours and then back to ambient temperature, to obtain silicone-treated powders.

### [Comparative Example 4] (Surface treatment with fatty acid)

For the powders of Examples 1 through 10 and Example 12, the same treatment as in Comparative Example 3 was done at the same processing amounts as in the Examples, except that isostearic acid (manufactured by NOF Corporation) was used instead, to obtain isostearic acid-treated powders.

### [Comparative Example 5] (Surface treatment with acylated amino acid)

For the powders of Examples 1 through 10 and Example 12, the surface treatment method described in JP-A-58-72512 was carried out at the same processing amounts as in the Examples, to obtain stearoyl glutamic acid-treated powders.

### [Comparative Example 6] (Surface treatment with alkyl silane)

For the powders of Examples 1 through 10 and Example 12, the surface treatment method described in JP-A-64-90111 was carried out, using the same amount for coating as in the Examples, to obtain octyltrimethoxysilane-treated powders.

The half ester oil-treated powders in accordance with the invention and the treated powders in Comparative Examples, as obtained as described above, were evaluated by the following test methods. The results are shown in Tables 1 through 3.

### (Adhesion test)

About 100 mg of a surface-treated powder was attached on a sponge puff. An area of 5 cm × 5 cm is marked on the forearms of 10 panelists, on which the powder on the sponge puff was applied on the area at a load of 50 g/cm². The weight of the sponge puff after applying was divided by the amount of the powder first attached to the puff, to determine the amount of the powder adhered on skin. The procedure was repeated five times, to determine the average. A larger value indicates better adhesion to skin.

### (Test of sliding property (aesthetic feel))

A surface-treated powder was applied under a condition of 1 mg/cm² on a collagen paper (manufactured by Idemitsu Petrochemical Co., Ltd.; trade name of Supplale) on a piece of 8 cm × 5 cm dimension, which was then mounted on a tester for measuring the reciprocating dynamic friction coefficient (manufactured by Kato Tech Co., Ltd.), while the collagen paper without application was also mounted on the tester. At a load of 50 g/cm², further, a reciprocating motion was enforced five times, to determine the mean of the values of the dynamic friction coefficient (MIU). A smaller value indicates better sliding property.

### (Dispersibility test)

### 1. Measuring gloss value (in case of titanium mica as extender pigment)

A surface-treated powder was applied under a condition of 1.4 mg/cm² on a collagen paper (manufactured by Idemitsu Petrochemical Co., Ltd.; trade name of Supplale) on a piece of 8 cm x 5 cm dimension. The gloss value of the applied face at 45 ° - 45 ° was determined with a deformation gloss meter (Nippon Denshoku Industries Co., Ltd.; VGS-300A). A larger value indicates better dispersibility.

### 2. Opacity (in case of titanium dioxide)

10 g of a surface-treated titanium dioxide and 0.5 g of carbon black (Mitsubishi Chemical Co. , Ltd.) were mixed with 89.5 g of talc JA-46R (manufactured by Asada Milling Co., Ltd.). The resulting mixture was ground with an atomizer. The W value (whitening degree) of each surface-treated powder sample was measured with a color difference meter (Nippon Denshoku Industries Co., Ltd.: SZ-Σ90). A larger value indicates better dispersibility.

### 3. Coloring ability (in case of iron oxide)

2 g of each of surface-treated yellow iron oxide, red iron oxide and black iron oxide and 98.0 g of talc JA-46R (manufactured by Asada Milling Co., Ltd.) were mixed together and ground with an atomizer. The C* value (chroma) of each sample was measured with a color difference meter (manufactured by Nippon Denshoku Industries Co., Ltd. ; SZ-Σ90). In case of yellow iron oxide and red iron oxide, a larger value indicates better dispersibility. In case of black iron oxide, a smaller value indicates better dispersibility.

### 4. Measurement of in-vitro SPF value (in case of ultrafine titanium dioxide and ultrafine zinc oxide)

A surface-treated powder sample weighed at 1 g was added to and mixed thoroughly with a mixture oil of 3 g of acryl silicone oil (Shin-etsu Chemical Co., Ltd.: KP545), 3 g of an ester oil (manufactured by Nisshin Oillio Group: Cosmol 525) and 3 g of squalane. The resulting mixture was dispersed once with a Hoover muller under a condition of 25 rotations (load of 22.68 kg). The in-vitro SPF value of the resulting dispersion was measured with an SPF analyzer (OPTOMETRICS, USA). A larger value indicates better dispersibility.

### 5. Moist feel

10 panelists touched the individual surface-treated powders. The panelists were engaged to do other work, while the powders remained on their hands. Subsequently, a questionnaire was taken about the presence or absence of a moist feel on their hands. When half or more of the panelists made a reply of the presence of moist feel about the powder, the powder was determined as having moist feel.

**Table 1 (Results of evaluation of extender pigment and titanium mica)**

| | Adhesion | Sliding property | dispersibility | Moist feel |
|---|---|---|---|---|
| Sericite of Ex. 1 | 0.73 | 2.16 | 56.3 | presence |
| Mica of Ex. 2 | 0.68 | 1.88 | 65.4 | presence |
| Talc of Ex. 3 | 0.61 | 2.55 | 51.0 | presence |
| Titanium mica of Ex. 10 | 0.80 | 4.87 | 78.3 | presence |
| Powder La Vie of Ex. 12 | 0.87 | 2.36 | 58.9 | presence |
| Sericite of Comp. Ex. 1 | 0.53 | 2.35 | 49.5 | presence |
| Mica of Comp. Ex. 1 | 0.36 | 2.97 | 60.3 | presence |
| Talc of Comp. Ex. 1 | 0.35 | 3.65 | 43.2 | presence |
| Titanium mica of Comp. Ex. 1 | 0.42 | 5.96 | 68.4 | presence |
| Powder La Vie of Comp. Ex. 1 | 0.55 | 2.97 | 48.6 | absence |
| Sericite of Comp. Ex. 2 | 0.61 | 2.27 | 52.3 | presence |
| Mica of Comp. Ex. 2 | 0.42 | 2.75 | 61.5 | presence |
| Talc of Comp. Ex. 2 | 0.46 | 3.28 | 45.0 | presence |
| Titanium mica of Comp. Ex. 2 | 0.63 | 5.66 | 70.7 | presence |
| Powder La Vie of Comp. Ex. 2 | 0.58 | 3.05 | 50.1 | absence |
| Sericite of Comp. Ex. 3 | 0.28 | 3.66 | 35.3 | absence |
| Mica of Comp. Ex. 3 | 0.26 | 3.22 | 38.7 | absence |
| Talc of Comp. Ex. 3 | 0.31 | 3.53 | 30.9 | absence |
| Titanium mica of Comp. Ex. 3 | 0.40 | 5.98 | 54.1 | absence |
| Powder La Vie of Comp. Ex. 3 | 0.45 | 3.16 | 45.6 | absence |
| Sericite of Comp. Ex. 4 | 0.68 | 3.25 | 49.1 | absence |
| Mica of Comp. Ex. 4 | 0.60 | 3.01 | 60.5 | absence |
| Talc of Comp. Ex. 4 | 0.52 | 2.98 | 46.7 | absence |
| Titanium mica of Comp. Ex. 4 | 0.76 | 5.23 | 72.2 | absence |
| Powder La Vie of Comp. Ex. 4 | 0.40 | 3.33 | 47.9 | absence |
| Sericite of Comp. Ex. 5 | 0.61 | 3.35 | 38.4 | presence |
| Mica of Comp. Ex. 5 | 0.56 | 3.03 | 39.9 | presence |
| Talc of Comp. Ex. 5 | 0.48 | 3.25 | 35.6 | presence |
| Titanium mica of Comp. Ex. 5 | 0.72 | 5.82 | 58.5 | absence |
| Powder La Vie of Comp. Ex. 5 | 0.50 | 3.07 | 48.0 | absence |
| Sericite of Comp. Ex. 6 | 0.58 | 2.68 | 54.3 | absence |
| Mica of Comp. Ex. 6 | 0.42 | 2.33 | 60.2 | absence |
| Talc of Comp. Ex. 6 | 0.43 | 2.85 | 48.6 | absence |
| Titanium mica of Comp. Ex. 6 | 0.58 | 4.97 | 75.9 | absence |
| Powder La Vie of Comp. Ex. | 0.43 | 3.22 | 45.2 | absence |

**Table 2 (Results of evaluation of titanium dioxide and iron oxide)**

| | Adhesion | Sliding property | dispersibility | Moist feel |
|---|---|---|---|---|
| Titanium dioxide of Ex. 4 | 0.52 | 2.86 | 65.3 | presence |
| Yellow iron oxide of Ex. 5 | 0.57 | 2.56 | 25.7 | presence |
| Red iron oxide of Ex. 6 | 0.61 | 2.68 | 33.6 | presence |
| Black iron oxide of Ex. 7 | 0.65 | 2.43 | 1.6 | presence |
| Titanium dioxide of Comp. Ex. | 0.41 | 3.87 | 45.3 | presence |
| Yellow iron oxide of Comp. Ex. | 0.49 | 3.66 | 18.5 | presence |
| Red iron oxide of Comp. Ex. 1 | 0.52 | 3.96 | 25.2 | presence |
| Black iron oxide of Comp. Ex. | 0.53 | 3.34 | 3.5 | presence |
| Titanium dioxide of Comp. Ex. 2 | 0.42 | 3.72 | 48.7 | presence |
| Yellow iron oxide of Comp. Ex. 2 | 0.50 | 3.59 | 19.7 | presence |
| Red iron oxide of Comp. Ex. 2 | 0.51 | 3.84 | 27.9 | presence |
| Black 2 iron oxide of Comp. Ex. | 0.55 | 3.29 | 2.6 | presence |
| Titanium dioxide of Comp. Ex. 3 | 0.25 | 4.35 | 32.2 | absence |
| Yellow iron oxide of Comp. Ex. 3 | 0.23 | 4.86 | 15.8 | absence |
| Red iron oxide of Comp. Ex. 3 | 0.22 | 4.23 | 25.0 | absence |
| Black iron oxide of Comp. Ex. 3 | 0.35 | 3.66 | 4.2 | absence |
| Titanium dioxide of Comp. Ex. 4 | 0.45 | 3.32 | 54.7 | absence |
| Yellow iron oxide of Comp. Ex. 4 | 0.49 | 3.45 | 22.2 | absence |
| Red iron oxide of Comp. Ex. 4 | 0.55 | 3.87 | 31.5 | absence |
| Black iron oxide of Comp. Ex.4 | 0.51 | 3.41 | 2.3 | absence |
| Titanium dioxide of Comp. Ex. 5 | 0.34 | 4.01 | 42.7 | presence |
| Yellow iron oxide of Comp. Ex. 5 | 0.36 | 4.28 | 18.7 | presence |
| Red iron oxide of Comp. Ex. 5 | 0.36 | 4.06 | 28.8 | presence |
| Black iron oxide of Comp. Ex. | 0.38 | 3.43 | 3.4 | presence |
| Titanium dioxide of Comp. Ex. 6 | 0.33 | 3.11 | 50.7 | absence |
| Yellow iron oxide of Comp. Ex. 6 | 0.31 | 2.85 | 23.3 | absence |
| Red iron oxide of Comp. Ex. 6 | 0.36 | 2.95 | 30.5 | absence |
| Black iron oxide of Comp. Ex. 6 | 0.39 | 2.81 | 1.9 | absence |

**Table 3 (Results of evaluation of ultrafine titanium dioxide and ultrafine zinc oxide)**

| | Adhesion | Sliding property | dispersibility | Moist feel |
|---|---|---|---|---|
| Ultrafine titanium dioxide of Ex. 8 | 0.43 | 2.36 | 28.1 | presence |
| Ultrafine zinc oxide of Ex. 9 | 0.41 | 2.63 | 13.2 | presence |
| Ultrafine titanium dioxide of Ex. 11 | 0.40 | 2.98 | 22.3 | presence |
| Ultrafine titanium dioxide of Comp. Ex. 1 | 0.31 | 3.15 | 12.5 | presence |
| Ultrafine zinc oxide of Comp. Ex. 1 | 0.30 | 3.32 | 4.3 | presence |
| Ultrafine titanium dioxide of Comp. Ex. 2 | 0.32 | 2.98 | 15.6 | presence |
| Ultrafine zinc oxide of Comp. Ex. 2 | 0.32 | 3.25 | 5.7 | presence |
| Ultrafine titanium dioxide of Comp. Ex. 3 | 0.17 | 2.94 | 21.0 | absence |
| Ultrafine zinc oxide of Comp. Ex. 3 | 0.11 | 3.49 | 7.5 | absence |
| Ultrafine titanium dioxide of Comp. Ex. 4 | 0.37 | 3.25 | 20.8 | absence |
| Ultrafine zinc oxide of Comp. Ex. 4 | 0.35 | 3.55 | 7.1 | absence |
| Ultrafine titanium dioxide of Comp. Ex. 5 | 0.26 | 3.88 | 19.3 | absence |
| Ultrafine zinc oxide of Comp. Ex. 5 | 0.28 | 3.61 | 7.7 | absence |
| Ultrafine titanium dioxide of Comp. Ex. 6 | 0.29 | 2.52 | 25.5 | absence |
| Ultrafine zinc oxide of Comp. Ex. 6 | 0.33 | 2.78 | 11.6 | absence |

As apparently shown in Tables 1, 2 and 3, the surface-treated powders of the invention have great adhesion, excellent spreadability, and high dispersion as well as moist feel.

Then, the treated powders of the invention were blended in individual cosmetics to verify the effects thereof, compared with treated powders of the past related art.

### [Example 13] (Two-way powder foundation)

A 2-way powder foundation of the composition shown in Table 4 was produced by the following method.

**Table 4**

| | | |
|---|---|---|
| 1. | Red iron oxide (Example and Comparative Examples): | 0.9 |
| 2. | Yellow iron oxide (Example and Comparative Examples): | 2.5 |
| 3. | Black iron oxide (Example and Comparative Examples): | 0.3 |
| 4. | Titanium dioxide (Example and Comparative Examples): | 12.0 |
| 5. | Sericite (Example and Comparative Examples): | 25.0 |
| 6. | Talc (Example and Comparative Examples): | qs. |
| 7. | Mica (Example and Comparative Examples): | 6.0 |
| 8. | Glyceryl trioctanoate: | 5.7 |
| 9. | Fluid paraffin: | 2.5 |
| 10. | Methylphenylpolysiloxane: | 5.3 |
| 11. | Preservative: | appropriate amount |
| 12. | Fragrance: | appropriate amount |

### (Production Process)

The components 1 through 7 were mixed together and uniformly ground.

The components 8 through 12 were added to the mixture (1), for grinding and pressed to obtain a 2-way powder foundation. The resulting foundations were evaluated by 50 panelists concerning adhesion to skin, feel on use (texture), and moist feel according to the following rating scale. The mean of the evaluated scores was determined. Concerning dispersibility, the surface color of the foundation was measured to determine the chroma value.

### Evaluated scores:

5: good
4: more or less good
3: ordinary
2: slightly poor
1: poor.

The results are as shown in Table 5.

**Table 5**

| | Exam-ple | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Adhesion | 4.2 | 3.3 | 3.5 | 2.3 | 3.9 | 3.7 | 3.3 |
| Feel on use | 4.0 | 3.4 | 3.4 | 2.0 | 3.5 | 3.7 | 3.8 |
| Moist feel | 4.5 | 4.0 | 4.2 | 1.6 | 3.6 | 4.2 | 2.3 |
| Dispersibility | 42.6 | 30.2 | 33.5 | 33.1 | 39.5 | 38.7 | 40.7 |

As apparently shown in Table 5, the foundation blended with the treated powder of the invention as obtained in the Example has great adhesion to skin, good spreadability on skin, very good dispersibility in general oils such as ester oils and triglyceride, and silicone-series oils, and moist feel, compared with the foundations blended with the treated powders of the related art. Additionally, the foundation retained well on skin and was stable over time.

### [Example 14] (emulsion-type foundation)

An emulsion type foundation of the composition shown in Table 6 was prepared by the following method.

**Table 6**

| | | |
|---|---|---|
| 1. | Decamethylcyclopentasiloxane | 20.0 |
| 2. | Dimethylpolysiloxane 6 cs | 3.0 |
| 3. | Lauroylglutamic acid dioctyl dodecyl | 3.0 |
| 4. | Squalane | 5.0 |
| 5. | Isononaic acid isotridecyl | 5.0 |
| 6. | Dimethylpolysiloxane polyoxyalkylene polymer (HLB= 4.0) | 4.5 |
| 7. | Red iron oxide (Example and Comparative Examples) | 1.6 |
| 8. | Yellow iron oxide (Example and Comparative Examples) | 2.5 |
| 9. | Black iron oxide (Example and Comparative Examples) | 0.1 |
| 10. | Titanium dioxide (Example and Comparative Examples) | 8.5 |
| 11. | Talc (Example and Comparative Examples) | 2.5 |
| 12. | Ethanol | 7.0 |
| 13. | Dipropylene glycol | 5.0 |
| 14. | Sodium chloride | 2.0 |
| 15. | Distilled water | qs. |
| 16. | Preservative | appropriate amount |
| 17. | Fragrance | appropriate amount |

### (Production Process)

(1) The components 1 through 6 were mixed together and dissolved together, to which the components 7 through 11 were added for uniform dispersion.
(2) The components 12 through 16 were dissolved under heating.
(3) The component mixture obtained above in (2) was gradually added to the component mixture obtained above in (1), for emulsification and subsequent cooling. Then, component 17 was added to the resulting cooled mixture to obtain a foundation. In the same manner as in Example 13, 50 panelists evaluated the adhesion to skin, feel on use (texture), and moist feel of the resulting foundations, to determine the mean of the evaluated scores. Concerning dispersibility, the liquid color of a foundation was measured to determine the chroma value. The results are shown in Table 7.

**Table 7**

| | Exam-ple | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Adhesion | 4.0 | 3.0 | 3.4 | 3.3 | 3.9 | 3.7 | 3.7 |
| Feel on use | 4.1 | 3.4 | 3.5 | 2.5 | 3.5 | 3.4 | 3.5 |
| Moist feel | 4.5 | 4.1 | 4.2 | 3.2 | 3.7 | 4.4 | 3.5 |
| Dispersibility | 46.3 | 38.8 | 41.5 | 37.2 | 39.5 | 42.6 | 43.0 |

As apparently shown in Table 7, the foundation blended with the treated powder of the invention as obtained in the Example has great adhesion to skin, good spreadability on skin, very good dispersibility in general oils such as ester oils, triglyceride, and silicone-series oils, and moist feel, compared with the foundations blended with the treated powders of the past related art. Additionally, the foundation retained well on skin and was stable over time.

### [Example 15] (eye shadow)

An eye shadow of the composition shown in Table 8 was prepared by the following method.

**Table 8**

| | | |
|---|---|---|
| 1. | Mica (Example and Comparative Examples) | 45.0 |
| 2. | Talc (Example and Comparative Examples) | qs. |
| 3. | Titanium mica (Example and Comparative Examples) | 10.5 |
| 4. | Titanium dioxide (Example and Comparative Examples) | 7.5 |
| 5. | Malic acid diisostearyl | 3.0 |
| 6. | Liquid lanoline | 2.5 |
| 7. | Monoisostearic acid sorbitan | 1.0 |
| 8. | Ceresin wax | 3.5 |
| 9. | Dimer acid diisopropyl | 6.5 |
| 10. | Fragrance | appropriate amount |

### (Production Process)

(1) The components 1 through 4 were mixed together and dissolved together.
(2) To the component mixture above in (1) were added the components 5 through 10 for grinding and press molding, to obtain an eye shadow.

In the same manner as in Example 13, 50 panelists evaluated the adhesion to skin, feel on use (texture), and moist feel of the resulting eye shadow products, to determine the mean of the evaluated scores. Concerning dispersibility, the surface gloss of an eye shadow was measured. The results are shown in Table 9.

**Table 9**

| | Example | Comp. Ex. 1 | Comp. Ex. 2 | Comp. Ex. 3 | Comp. Ex. 4 | Comp. Ex.5 | Comp. Ex. 6 |
|---|---|---|---|---|---|---|---|
| Adhesion | 4.8 | 3.4 | 3.7 | 2.5 | 3.7 | 4.2 | 3.6 |
| Feel on use | 4.7 | 3.8 | 3.9 | 2.1 | 3.9 | 3.6 | 4.1 |
| Moist feel | 4.7 | 4.0 | 4.1 | 2.0 | 3.5 | 4.2 | 2.5 |
| Dispersibility | 57.3 | 33.2 | 39.7 | 35.8 | 52.7 | 45.1 | 55.0 |

As apparently shown in Table 9, the eye shadow blended with the treated powder of the invention as obtained in the Example has great adhesion to skin, good spreadability on skin, very good dispersibility in general oils such as ester oils, triglyceride, and silicone-series oils, and moist feel, compared with the eye shadow products blended with the treated powders of the related art. Additionally, the eye shadow retained well on skin and was stable over time.

### [Example 16] (Emollient cream)

An emollient cream of the composition shown in Table 10 was prepared by the following method.

**Table 10**

| | | |
|---|---|---|
| 1. | Dimethylpolysiloxane 6cs | 7.0 |
| 2. | Methylphenylpolysiloxane | 5.0 |
| 3. | Squalane | 5.0 |
| 4. | Dioctanoic acid neopentyl glycol | 4.5 |
| 5. | Polyether-modified silicone (HLB = 4.5) | 3.5 |
| 6. | Talc (Example and Comparative Examples) | 5.0 |
| 7. | Red iron oxide (Example and Comparative Examples) | 0.1 |
| 8. | Magnesium sulfate | 0.7 |
| 9. | Glycerin | 10.0 |
| 10. | Preservative | appropriate amount. |
| 11. | Distilled water | qs. |
| 12. | Fragrance | appropriate amount |

### (Production Process)

(1) The components 1 through 5 were mixed together and dissolved together, to which the components 6 and 7 were added for uniform dispersion.
(2) The components 8 through 11 are dissolved under heating.
(3) The component mixture obtained above in (2) was gradually added to the component mixture obtained above in (1), for emulsification and subsequent cooling. Then, component 12 was added to the resulting cooled mixture to obtain an emollient cream.

In the same manner as in Example 13, 50 panelists evaluated the adhesion to skin, feel on use (texture), and moist feel of the resulting creams, to determine the mean of the evaluated scores. Concerning dispersibility, the liquid color of a cream was measured to determine the chroma value. The results are shown in Table 11.

**Table 11**

| | Example | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Adhesion | 4.5 | 3.7 | 4.1 | 3.9 | 4.0 | 4.3 | 4.2 |
| Feel on use | 4.7 | 3.7 | 3.8 | 3.5 | 4.3 | 4.1 | 4.2 |
| Moist feel | 4.6 | 4.0 | 4.2 | 3.0 | 3.6 | 3.5 | 3.1 |
| Dispersibility | 3.9 | 2.8 | 3.0 | 3.0 | 3.2 | 3.4 | 3.8 |

As apparently shown in Table 9, the emollient cream blended with the treated powder of the invention as obtained in the Example has great adhesion to skin, good spreadability on skin, very good dispersibility in general oils such as ester oils, triglyceride, and silicone-series oils, and moist feel, compared with the emollient creams blended with the treated powders of the related art. Additionally, the cream was retained well on skin and was stable over time.

### [Example 17] (Two-layer type sun screening lotion)

A sun screening lotion of the composition shown in Table 12 was prepared by the following method.

**Table 12**

| | | |
|---|---|---|
| 1. | Decamethylcyclopentasiloxane | 15.0 |
| 2. | Isononaic acid isooctyl | 5.0 |
| 3. | Fluorine-modified silicone (manufactured by Asahi Glass Co., Ltd.: FSL-300) | 6.0 |
| 4. | Perfluoropolyether (manufactured by Montefros Corporation: FOMBLIN HC-04) | 5.0 |
| 5. | Polyether-modified silicone (HLB = 3.5) | 4.5 |
| 6. | Ultrafine zinc oxide (Example and Comparative Examples) | 10.0 |
| 7. | Ultrafine titanium dioxide (Examples 8 and 11 and Comparative Examples) | 10.0 |
| 8. | Glycerin | 8.0 |
| 9. | Xantham Gum | 0.3 |
| 10. | Tetrasodium edetate | 0.1 |
| 11. | L-Ascorbate and phosphate ester magnesium salt | 2.5 |
| 12. | Sodium citrate | 1.0 |
| 13. | Distilled water | qs. |

### (Production Process)

(1) The components 1 through 5 were mixed together and dissolved together, to which the components 6 and 7 were added for uniform dispersion.
(2) The components 8 through 13 are dissolved under heating.
(3) The component mixture obtained above in (2) was gradually added to the component mixture obtained above in (1), for emulsification and subsequent cooling, to obtain a sun screening lotion. In the same manner as inExample 13, 50 panelists evaluated the adhesion to skin, feel on use (texture), and moist feel of the resulting lotions, to determine the mean of the evaluated scores. Concerning dispersibility, the in-vitro SPF value of a lotion was measured. The results are shown in Table 13.

**Table 13**

| | Example 8 in blend | Example 11 in blend | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|---|
| Adhesion | 4.5 | 4.5 | 3.8 | 3.9 | 3.9 | 4.0 | 4.0 | 4.2 |
| Feel on use | 4.6 | 4.3 | 3.5 | 3.9 | 3.5 | 4.3 | 3.6 | 4.1 |
| Moist feel | 4.8 | 4.2 | 4.2 | 4.3 | 3.5 | 3.4 | 4.1 | 3.9 |
| Dispersibility | 52.1 | 50.9 | 33.6 | 39.5 | 46.7 | 37.5 | 39.0 | 48.8 |

As apparently shown in Table 13, the sun screening lotions blended with the treated powders of the invention as obtained in the Examples have great adhesion to skin, good spreadability on skin, very great dispersibility in general oils such as ester oils, triglyceride, and silicone-series or fluorine-series oils and moist feel, compared with the sun screening lotions blended with the treated powders of the related art. Additionally, the lotions retained well on skin and were stable over time.

### [Example 18] (Lipstick)

A lipstick of the composition shown in Table 14 was prepared by the following method.

**Table 14**

| | | |
|---|---|---|
| 1. | Malic acid diisostearyl | 15.0 |
| 2. | Triisooctanoic acid glyceryl | qs. |
| 3. | Glutamic acid phytosterol octyldodecanol | 15.0 |
| 4. | Carnauba wax | 3.5 |
| 5. | Candelilla wax | 5.0 |
| 6. | Ceresin wax | 5.0 |
| 7. | Titanium dioxide (Example and Comparative Examples) | 7.0 |
| 8. | Red No. 201 (5 % of the surface was treated by the methods in Example 3 and the individual Comparative Examples) | 3.0 |
| 9. | Black iron oxide (Example and Comparative Examples) | 0.1 |
| 10. | Preservative | appropriate amount |

### (Production Process)

(1) The components 7 through 9 are added to the component 1, for kneading with a roller for uniform dispersion.
(2) The other components are heated and mixed and dissolved together, to which the component mixture above in (1) is added, for uniform dispersion with a homomixer.
(3) After deaeration, the resulting mixture was poured in a mold to prepare a lipstick in the form of a stick.

In the same manner as in Example 13, 50 panelists evaluated the adhesion to skin, feel on use (texture), and moist feel of the resulting lipsticks, to determine the mean of the evaluated scores. Concerning dispersibility, the apparent color of a lipstick was measured to determine the chroma value. The results are shown in Table 15.

**Table 15**

| | Example | Comp. Ex. 1 | Comp. Ex.2 | Comp. Ex. 3 | Comp. Ex. 4 | Comp. Ex. 5 | Comp. Ex. 6 |
|---|---|---|---|---|---|---|---|
| Adhesion | 4.3 | 3.2 | 3.6 | 3.6 | 4.2 | 4.0 | 4.0 |
| Feel on use | 4.3 | 3.4 | 3.7 | 3.5 | 3.9 | 4.0 | 4.2 |
| Moist feel | 4.3 | 3.9 | 4.1 | 3.2 | 3.7 | 4.1 | 3.6 |
| Dispersibility | 63.3 | 50.5 | 57.3 | 51.2 | 62.5 | 59.7 | 60.8 |

As apparently shown in Table 15, the lipstick blended with the treated powder of the invention as obtained in the Example has great adhesion to skin, good spreadability on skin, very good dispersibility in general oils such as ester oils and triglyceride, and moist feel, compared with the lipsticks blended with the treated powders of the related art. Additionally, the lipstick retained well on skin and was stable over time.

### [Example 19] (Rinse)

A rinse of the composition shown in Table 16 was prepared by the following method.

**Table 16**

| | | |
|---|---|---|
| 1. | Stearyltrimethylammonium chloride | 1.5 |
| 2. | Cetanol | 3.0 |
| 3. | Myristic acid octyl dodecyl | 2.5 |
| 4. | Talc (Example and Comparative Examples) | 1.5 |
| 5. | Distilled water | qs. |
| 6. | Preservative | appropriate amount |

### (Production Process)

(1) The components 1 through 4 are uniformly dissolved and mixed together.
(2) To a solution of the components 5 and 6 in uniform dissolution was added the component mixture above in (1) for emulsification, to obtain a rinse. In the same manner as in Example 13, 50 panelists evaluated the adhesion to skin, feel on use (texture), and moist feel of the resulting rinse products, to determine the mean of the evaluated scores. Concerning dispersibility, the color of hair gloss was measured. The results are shown in Table 17.

**Table 17**

| | Example | Comp. Ex. 1 | Comp. Ex. 2 | Comp. Ex.3 | Comp. Ex. 4 | Comp. Ex. 5 | Comp. Ex. 6 |
|---|---|---|---|---|---|---|---|
| Adhesion | 4.1 | 3.5 | 3.6 | 3.2 | 3.8 | 3.7 | 3.6 |
| Feel on use | 4.0 | 3.2 | 3.4 | 3.1 | 3.5 | 3.6 | 3.5 |
| Moist feel | 4.5 | 3.8 | 4.0 | 3.1 | 3.4 | 3.5 | 3.4 |
| Dispersibility | 16.5 | 14.5 | 15.3 | 15.8 | 16.2 | 15.9 | 16.4 |

As apparently shown in Table 17, the rinse blended with the treated powder of the invention as obtained in the Example has great adhesion to skin, good spreadability on skin, very good dispersibility in general oils such as ester oils, triglyceride, and moist feel, compared with the rinse products blended with the treated powders of the related art. Additionally, the rinse retained well on skin and was stable over time.

### [Example 20] (Lip gloss)

A lip gloss of the composition shown in Table 18 was prepared by the following method.

**Table 18**

| | | |
|---|---|---|
| 1. | Palmitic acid dextrin | 8.0 |
| 2. | Tri(caprylic acid - capric acid) glycerin | 45.0 |
| 3. | Squalane | 29.5 |
| 4. | Methylphenylpolysiloxane | 15.0 |
| 5. | Titanium mica (Example and Comparative Examples) | 2.5 |

### (Production Process)

(1) The components 1 through 4 are dissolved together uniformly under heating.
(2) The component 5 was added to the component mixture above in (1). The resulting mixture was mixed together uniformly and then cooled, to obtain a lip gloss.

In the same manner as in Example 13, 50 panelists evaluated the adhesion to skin, feel on use (texture), and moist feel of the resulting lip gloss products, to determine the mean of the evaluated scores. Concerning dispersibility, the gloss of a lip gloss was measured. The results are shown in Table 19.

**Table 19**

| | Example | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Adhesion | 4.6 | 4.0 | 4.1 | 3.6 | 4.2 | 4.0 | 4.1 |
| Feel on use | 4.7 | 3.2 | 3.6 | 3.1 | 4.0 | 4.2 | 4.3 |
| Moist feel | 4.9 | 4.0 | 4.4 | 2.6 | 4.2 | 4.5 | 3.8 |
| Dispersibility | 13.7 | 8.2 | 11.7 | 9.2 | 13.0 | 11.5 | 12.8 |

As apparently shown in Table 19, the lip gloss blended with the treated powder of the invention as obtained in the Example has great adhesion to skin, good spreadability on skin, very good dispersibility in general oils such as ester oils, triglyceride, and silicone-series oils and moist feel, compared with the lip gloss products blended with the treated powders of the related art. Additionally, the lip gloss retained well on skin and was stable over time.

### [Example 21] (Eyeliner)

An eyeliner of the composition shown in Table 20 was prepared by the following method.

**Table 20**

| | | Blend (%) |
|---|---|---|
| 1. | Powder La Vie (Example and Comparative Examples) | 10.00 |
| 2. | Titanium dioxide (Example and Comparative Examples) | 3.00 |
| 3. | Black iron oxide (Example and Comparative Examples) | 3.50 |
| 4. | Organic bentonite | 0.50 |
| 5. | Light fluid isoparaffin | 67.10 |
| 6. | Carnauba wax | 4.50 |
| 7. | Beeswax | 1.00 |
| 8. | Microcrystalline wax | 11.00 |
| 9. | Vaseline | 2.00 |
| 10. | Anti-oxidant | 0.20 |
| 11. | Preservative | 0.20 |

### (Production Process)

The components 1 through 4 and a part of the component 5 are mixed together for dispersion. To the resulting mixture are added the components 5 through 11 after dissolution under heating. The resulting mixture is kneaded together with a roll mill, and is again heated and dissolved, to which the remaining part of component 5 is added. The resulting mixture was cooled under agitation, to obtain an eyeliner. In the same manner as in Example 13, 50 panelists evaluated the adhesion to skin, feel on use (texture), and moist feel of the resulting eyeliner products, determine the mean of the evaluated scores. Concerning dispersibility, the gloss of an eye liner was measured. The results are shown in Table 21.

**Table 21**

| | Example Example | Comp. Ex. 1 | Comp. Ex. 2 | Comp. Ex. 3 | Comp. Ex. 4 | Comp. Ex. 5 | Comp. Ex. 6 |
|---|---|---|---|---|---|---|---|
| Adhesion | 4.8 | 3.3 | 4.0 | 3.7 | 3.2 | 3.5 | 3.8 |
| Feel on use | 4.8 | 3.8 | 3.8 | 3.3 | 3.8 | 3.2 | 3.3 |
| Moist feel | 4.8 | 3.9 | 3.9 | 2.2 | 2.9 | 3.1 | 3.5 |
| Dispersibility | 4.7 | 2.5 | 1.7 | 1.9 | 2.5 | 2.7 | 3.0 |

As apparently shown in Table 21, the eye liner blended with the treated powder of the invention as obtained in the Example has great adhesion to skin, good spreadability on skin, very good dispersibility in general oils such as ester oils, triglyceride, and silicone-series oils, and moist feel, compared with the eye liners blended with the treated powders of the related art. Additionally, the eye liner was retained well on skin and was stable over time.

### INDUSTRIAL APPLICABILITY

As described above, the surface-treated powder of the invention and cosmetics blended with the surface-treated powder have great adhesion to skin and hair, good spreadability on skin and hair, very good dispersibility in general oils such as ester oils, triglyceride, silicone-series oils and fluorine-series oils and moist feel. Thus, the cosmetics blended with such surface-treated powder base can get great improvement in terms of feel on use, makeup retention, and product stability over time.

## Claims

1. A surface-treated powder coated with a half ester oil, wherein the half ester oil comprises an ester compound having 16 or more carbon atoms in total obtainable by reacting one or more alcohols having one to 36 carbon atoms with one or more carboxylic acids having one to 36 carbon atoms, and wherein the half ester oil has an acid value of 15 or more.

2. The surface-treated powder according to claim 1, wherein the alcohol is monovalent or polyvalent comprising at least one selected from the group consisting of linear carbons, branched carbons, saturated carbons, unsaturated carbons, alicyclic carbons and aromatic rings.

3. The surface-treated powder according to claim 1, wherein the carboxylic acid is monobasic acid or polybasic acid comprising at least one selected from the group consisting of linear carbons, branched carbons, saturated carbons, unsaturated carbons, alicyclic carbons and aromatic rings, or hydroxylic acid thereof.

4. The surface-treated powder according to claim 1, wherein the acid value of the half ester oil is 15 or more to 100 or less.

5. The surface-treated powder according to any one of claims 1 through 4, wherein the ratio by weight between a powder to be coated and the half ester oil is 100 : 0.1 to 50.

6. A cosmetic comprising a surface-treated powder according to any one of claims 1 through 5 at 0.1 to 100 % by weight.

7. A surface-treated powder coated with a half ester oil, wherein the half ester oil comprises an ester compound having 16 or more carbon atoms in total obtainable by reacting one or more alcohols having one to 34 carbon atoms with one or more carboxylic acids having one to 30 carbon atoms, and wherein the half ester oil has an acid value of 15 or more.

## Patentansprüche

1. Oberflächenbehandeltes Pulver, beschichtet mit einem Halbester-Öl, wobei das Halbester-Öl umfasst eine EsterVerbindung mit insgesamt 16 oder mehr Kohlenstoffatomen, erhältlich durch Reagieren eines oder mehrerer Alkohole, der bzw. die ein bis 36 Kohlenstoffatome besitzt bzw. besitzen, mit einer oder mehreren Carbonsäuren, die 1 bis 36 Kohlenstoffatome besitzt bzw. besitzen, und wobei das Halbester-Öl eine Säurezahl von 15 oder mehr besitzt.

2. Oberflächenbehandeltes Pulver nach Anspruch 1, wobei der Alkohol einwertig oder mehrwertig ist, umfassend mindestens eines, gewählt aus der Gruppe bestehend aus linearen Kohlenstoffen, verzweigten Kohlenstoffen, gesättigten Kohlenstoffen, ungesättigten Kohlenstoffen, alicyclischen Kohlenstoffen und aromatischen Ringen.

3. Oberflächenbehandeltes Pulver nach Anspruch 1, wobei die Carbonsäure eine einbasige Säure oder mehrbasige Säure ist, umfassend mindestens eines, gewählt aus der Gruppe bestehend aus linearen Kohlenstoffen, verzweigten Kohlenstoffen, gesättigten Kohlenstoffen, ungesättigten Kohlenstoffen, alicyclischen Kohlenstoffen und aromatischen Ringen, oder Hydroxysäuren davon.

4. Oberflächenbehandeltes Pulver nach Anspruch 1, wobei die Säurezahl des Halbester-Öls 15 oder mehr bis 100 oder weniger ist.

5. Oberflächenbehandeltes Pulver nach einem der Ansprüche 1 bis 4, wobei das Gewichtsverhältnis zwischen einem zu beschichtenden Pulver und dem Halbester-Öl beträgt 100 : 0.1 bis 50.

6. Kosmetik, umfassend ein oberflächenbehandeltes Pulver nach einem der Ansprüche 1 bis 5 zu 0,1 bis 100 Gewichtsprozent.

7. Oberflächenbehandeltes Pulver, beschichtet mit einem Halbester-Öl, wobei das Halbester-Öl umfasst eine Esterverbindung mit insgesamt 16 oder mehr Kohlenstoffatomen, erhältlich durch Reagieren eines oder mehrerer Alkohole, der bzw. die ein bis 34 Kohlenstoffatome besitzt bzw. besitzen, mit einer oder mehreren Carbonsäuren, die 1 bis 30 Kohlenstoffatome besitzt bzw. besitzen, und wobei das Halbester-Öl eine Säurezahl von 15 oder mehr besitzt.

## Revendications

1. Poudre traitée en surface enrobée d'une huile d'hémiester, dans laquelle l'huile d'hémiester comprend un ester ayant au moins 16 atomes de carbone au total, pouvant être obtenu en faisant réagir un ou plusieurs alcools ayant un à 36 atomes de carbone avec un ou plusieurs acides carboxyliques ayant un à 36 atomes de carbone, et dans laquelle l'huile d'hémiester a un indice d'acide de 15 ou plus.

2. Poudre traitée en surface selon la revendication 1, dans laquelle l'alcool est un alcool monovalent ou polyvalent comprenant au moins un membre du groupe formé par ceux ayant des carbones linéaires, des carbones ramifiés, des carbones saturés, des carbones insaturés, des carbones alicycliques et des noyaux aromatiques.

3. Poudre traitée en surface selon la revendication 1, dans laquelle l'acide carboxylique est un monoacide ou un polyacide comprenant au moins un membre du groupe formé par ceux ayant des carbones linéaires, des carbones ramifiés, des carbones saturés, des carbones insaturés, des carbones alicycliques et des noyaux aromatiques, ou un acide hydroxylique de celui-ci.

4. Poudre traitée en surface selon la revendication 1, dans laquelle l'indice d'acide de l'huile d'hémiester est de 15 ou plus à 100 ou moins.

5. Poudre traitée en surface selon l'une quelconque des revendications 1 à 4, dans laquelle le rapport en poids entre une poudre à enrober et l'huile d'hémiester est de 100 : 0,1 à 50.

6. Produit cosmétique comprenant une poudre traitée en surface selon l'une quelconque des revendications 1 à 5 en une quantité de 0,1 à 100 % en poids.

7. Poudre traitée en surface enrobée d'une huile d'hémiester, dans laquelle l'huile d'hémiester comprend un ester ayant au moins 16 atomes de carbone au total, pouvant être obtenu en faisant réagir un ou plusieurs alcools ayant un à 34 atomes de carbone avec un ou plusieurs acides carboxyliques ayant un à 30 atomes de carbone, et dans laquelle l'huile d'hémiester a un indice d'acide de 15 ou plus.
